# EUROPEAN PATENT SPECIFICATION

(11) **EP 3 463 329 B1**
(45) Date of publication and mention of the grant of the patent: **12.02.2025**
(21) Application number: 17803433.6
(22) Date of filing: 23.05.2017
(51) Int. Cl.: A61K 31/194, A61K 31/282, A61K 31/454, A61K 36/484, A61K 36/539, A61K 36/725, A61K 39/39, A61K 39/00, A61P 35/00, A61K 36/65

(54) **IMPROVED THERAPEUTIC INDEX OF ANTI-IMMUNE CHECKPOINT INHIBITORS USING COMBINATION THERAPY COMPRISING A PHY906 EXTRACT OR A SCUTELLARIA BAICALENSIS GEORGI (S) EXTRACT**
VERBESSERTER THERAPEUTISCHER INDEX VON ANTI-IMMUN-CHECKPOINT-INHIBITOREN UNTER VERWENDUNG EINER KOMBINATIONSTHERAPIE MIT EINEM PHY906-EXTRAKT ODER EINEM SCUTELLARIA BAICALENSIS GEORGI (S) EXTRAKT
AMÉLIORATION DE L'INDICE THÉRAPEUTIQUE D'INHIBITEURS DES POINTS DE CONTRÔLE ANTI-IMMUNITAIRE PAR UTILISATION D'UN TRAITEMENT D'ASSOCIATION COMPRENANT UN EXTRAIT DE PHY906 OU UN EXTRAIT DE SCUTELLARIA BAICALENSIS GEORGI (S)

(30) Priority: 23.05.2016 US 201662340164 P
(43) Date of publication of application: 10.04.2019
(73) Proprietor: Yale University, New Haven, CT 06511 (US)
(72) Inventor: CHENG, Yung-Chi, Woodbridge Connecticut 06525 (US); CHEN, Lieping, Hamden Connecticut 06514 (US)
(74) Representative: Grünecker Patent- und Rechtsanwälte PartG mbB
(86) International application number: PCT/US2017/034025
(87) International publication number: WO 2017/205389

(56) References cited:
- EP-A1- 3 012 270
- WO-A1-2014/082083
- WO-A1-2015/023710
- WO-A1-2016/044207
- WO-A2-2006/053049
- WO-A2-2007/041276
- TERESA KIM ET AL: "Combining targeted therapy and immune checkpoint inhibitors in the treatment of metastatic melanoma", CANCER BIOLOGY & MEDICINE, TIANJIN YIKE DAXUE FUSHU ZHONGLIU YIYUAN,TIANJIN MEDICAL UNIVERSITY CANCER INSTITUTE AND HOSPITAL, CN, vol. 11, no. 4, 1 December 2014 (2014-12-01), pages 237 - 246, XP002759549, ISSN: 2095-3941, DOI: 10.7497/J.ISSN.2095-3941.2014.04.002
- CUI X ET AL: "Sunscreen skin moisturizing cream, useful for preventing skin inflammation and treating skin cancer, comprises e.g. colla corii asini, cinnamon, Scutellaria baicalensis, Sophora flower bud, mulberry bark, garden burnet, and grape seed", WPI / THOMSON,, vol. 2012, no. 46, 11 April 2012 (2012-04-11), XP002740440
- GIM HUIJIN ET AL: "Apoptosis of Human Bladder Cancer Cells by an Ethanolic Extract of Scutellaria Baicalensis GEORGI Via Caspase and MAPK Signaling Pathways", KOREAN JOURNAL ORIENTAL PHYSIOLOGY & PATHOLOGY, KOREAN SOCIETY OF ORIENTAL PATHOLOGY, KOREA, vol. 30, no. 2, 25 April 2016 (2016-04-25), pages 131 - 136, XP053034578, ISSN: 1738-7698, DOI: 10.15188/KJOPP.2016.04.30.2.131
- PARK HYEON SOO ET AL: "KoreanScutellaria baicalensisGeorgi methanol extracts inhibits metastasis via the Forkhead Box M1 activity in hepatocellular carcinoma cells", JOURNAL OF ETHNOPHARMACOLOGY, ELSEVIER IRELAND LTD, IE, vol. 155, no. 1, 6 June 2014 (2014-06-06), pages 847 - 851, XP029009978, ISSN: 0378-8741, DOI: 10.1016/J.JEP.2014.05.053
- PARK K I ET AL: "Korean Scutellaria baicalensis water extract inhibits cell cycle G1/S transition by suppressing cyclin D1 expression and matrix-metalloproteinase-2 activity in human lung cancer cells", JOURNAL OF ETHNOPHARMACOLOGY, ELSEVIER IRELAND LTD, IE, vol. 133, no. 2, 27 January 2011 (2011-01-27), pages 634 - 641, XP027596147, ISSN: 0378-8741, [retrieved on 20110111], DOI: 10.1016/J.JEP.2010.10.057
- LAM W ET AL: "5584: PHY906(YIV-906), an adjuvant based on a 1800-year-old Chinese medicine, enhanced the anti-tumor activity of Immunecheckpointblockade therapy: Anti-PDL1, anti-PD1, anti-PD1/CTLA4 against melanoma", CANCER RESEARCH; AMERICAN ASSOCIATION FOR CANCER RESEARCH ANNUAL MEETING 2017, AMERICAN ASSOCIATION FOR CANCER RESEARCH, AACR ANNUAL MEETING 2018; APRIL 14-18, 2018; CHICAGO, IL; WASHINGTON, DC, USA, vol. 77, no. 13, Supplement 1, 1 July 2017 (2017-07-01) - 1 April 2017 (2017-04-01), pages 5584, XP009517126, ISSN: 1538-7445, DOI: 10.1158/1538-7445.AM2017-5584
- LAM W ET AL: "Chemotherapy: The four-herb Chinese medicine PHY906 reduces chemotherapy-induced gastrointestinal toxicity", SCIENCE TRANSLATIONAL MEDICINE, AMERICAN ASSOCIATION FOR THE ADVANCEMENT OF SCIENCE (A A A S), US, vol. 2, no. 45, 18 August 2010 (2010-08-18), pages 45ra59, XP009517132, ISSN: 1946-6234, DOI: 10.1126/SCITRANSLMED.3001270
- SHWU-HUEY LIU ET AL: "Old formula, new Rx: The journey of PHY906 as cancer adjuvant therapy", JOURNAL OF ETHNOPHARMACOLOGY, ELSEVIER IRELAND LTD, IE, vol. 140, no. 3, 25 January 2012 (2012-01-25), pages 614 - 623, XP028474751, ISSN: 0378-8741, [retrieved on 20120203], DOI: 10.1016/J.JEP.2012.01.047

## Description

### CROSS-REFERENCE TO RELATED APPLICATIONS

This application claims priority under 35 U.S.C. § 119(e) to U.S. Provisional Application No. 62/340,164 filed May 23, 2016.

### STATEMENT REGARDING FEDERALLY SPONSORED RESEARCH OR DEVELOPMENT

This invention was made with government support under CA154295-01A1 awarded by National Institutes of Health (NIH). The government has certain rights in the invention.

### BACKGROUND OF THE INVENTION

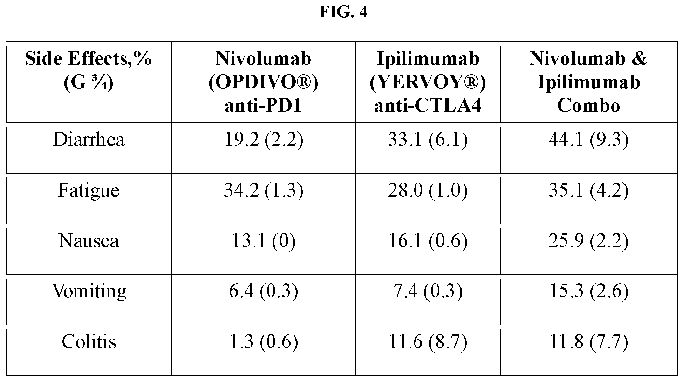

Many tumors can evade immune system responses by upregulating co-inhibitory pathways, also known as immune checkpoints. The PDL-PD1 pathway is one such immune checkpoint. When cytotoxic T cells (CD8-positive) approach tumor cells, they secrete interferon-gamma (IFNγ), which upregulates PD-L1 protein expression on the surface of the tumor cell. Interaction between the PD-L1 proteins on the tumor cell membrane and PD1 protein on the T-cell membrane can inactive the T-cell killing action, sparing the tumor from being eliminated by the T-cell. Inhibiting the PDL-PD1 pathway has been shown to be effective for inhibiting tumor cell growth in non-small cell lung cancer, melanoma and renal cell carcinoma, for example. Unfortunately, U.S. FDA-approved PD1 and PD-L1 inhibitors (such as, but not limited to, pembrolizumab (KEYTRUDA^{®}), nivolumab (OPDIVO^{®}), durvalumab (MEDI4736), Atezolizumab (TECENTRIQ^{®}), and ipilumumab (YERVOY^{®})) are also known to cause nausea, diarrhea, fatigue, colitis and vomiting, particularly in combination with anti-CTLA4 (see, for example, FIG. 4). WO2015/023710 relates to CTLA4 blockade as potential biomarker indicative to predict whether a patient will respond to an androgen-targeted therapy. Kim, T., et al. (Cancer Bio. Med., Vol. 11 (2014), p. 237-46) reviews the potential synergy between targeted therapy of melanoma and immune checkpoint blockade (anti-CTLA4, anti-PD-1 and anti PD-L1).

PHY906 is an herbal mixture extract based on the Huang Qin Tang herbal mixture, which was first described in Chinese texts 1,800 years ago, and has been used to treat gastrointestinal symptoms. PHY906 is composed of four herbs: *Scutellaria baicalensis (S), Glycyrrhiza uralensis (G), Paeonia lactiflora* (P), and *Ziziphus jujuba (Z).* PHY906 is currently manufactured according to cGMP (current Good Manufacturing Practice). In clinical trials, PHY906 is shown to enhance the therapeutic index of chemotherapy against cancer.

There is thus an unmet need in the art for novel compositions and methods for the treatment of tumors through inhibition of the PDL-PD1 pathway. In certain embodiments, such methods should enhance the therapeutic index of the PDL-PD1 pathway inhibitors and not compromise the anti-cancer activity of the anti-immune checkpoint therapy. The present invention satisfies this unmet need.

### SUMMARY OF THE INVENTION

The present invention is defined by the appended claims.

The present invention includes a kit comprising one or more immune checkpoint inhibitors, and at least one herbal composition selected from the group consisting of: (a) an herbal extract of *Scutellaria baicalensis* (S); (b) the herbal extract PHY906, which comprises herbal extracts of *Scutellaria baicalensis (S), Glycyrrhiza uralensis (G), Paeonia lactiflora* (P), and *Ziziphus jujuba* (Z) in a 3 : 2 : 2 : 2 ratio of S, G, P and Z; (c) an herbal extract comprising an extract of S; and (d) an herbal extract comprising a combination of S, G, P and Z.

In certain embodiments of the kits of the invention (a) or (c) further comprise an herbal extract, derived from at least one herb selected from the group consisting of *Glycyrrhiza uralensis (G), Paeonia lactiflora* (P), and *Ziziphus jujuba (Z).*

In certain embodiments, the immune checkpoint inhibitor is selected from the group consisting of an anti-PD1, an anti-PD-L1 and an anti-CTLA4. In other embodiments, the immune checkpoint inhibitor is selected from the group consisting of Ipilimumab, Pembrolizumab, Nivolumab, Durvalumab and Atezolizumab.

In certain embodiments, the kit further comprises one or more pharmaceutically acceptable carriers.

The invention further provides means for treating cancer in a subject, the means comprising administering to the subject in need thereof a therapeutically effective amount of at least one herbal composition for the treatment of cancer as disclosed herein and one or more immune checkpoint inhibitors, wherein the cancer is at least one selected from the group consisting of melanoma, non-small cell lung cancer, renal cell carcinoma, liver cancer, colon cancer, urothelial bladder cancer and pancreatic cancer. In certain embodiments, the cancer is melanoma.

In certain embodiments, the immune checkpoint inhibitor is selected from the group consisting of an anti-PD1, an anti-PD-L1 and an anti-CTLA4. In other embodiments, the immune checkpoint inhibitor is selected from the group consisting of Ipilimumab, Pembrolizumab, Nivolumab, Durvalumab and Atezolizumab.

In certain embodiments, the therapeutically effective amount of the herbal composition minimizes or eliminates one or more of the immune checkpoint inhibitors' gastro-intestinal side effects selected from the group consisting of nausea, vomiting, fatigue, colitis and diarrhea.

In certain embodiments, the herbal composition is administered to the subject orally. In other embodiments, the herbal composition is administered to the subject orally in a form selected from the group consisting of a pill, tablet, capsule, soup, tea, concentrate, dragees, liquids, drops, and gelcaps.

In certain embodiments, the therapeutically effective amount of the herbal composition is 20 mg/day to 2 g/day. In other embodiments, the therapeutically effective amount of the herbal composition is 1,600 mg/day. In yet other embodiments, the herbal composition is administered twice daily.

In certain embodiments, the herbal composition is administered twice daily for one to two weeks, followed by a suspension of treatment for at least one week.

In certain embodiments, the herbal composition is administered at a time selected from prior to, simultaneously with and after administration of the one or more immune checkpoint inhibitors to the subject.

In certain embodiments, the subject is a mammal. In other embodiments, the mammal is a human.

### BRIEF DESCRIPTION OF THE DRAWINGS

The following detailed description of specific embodiments of the invention will be better understood when read in conjunction with the appended drawings. For the purpose of illustrating the invention, there are shown in the drawings specific embodiments. It should be understood, however, that the invention is not limited to the precise arrangements and instrumentalities of the embodiments shown in the drawings.

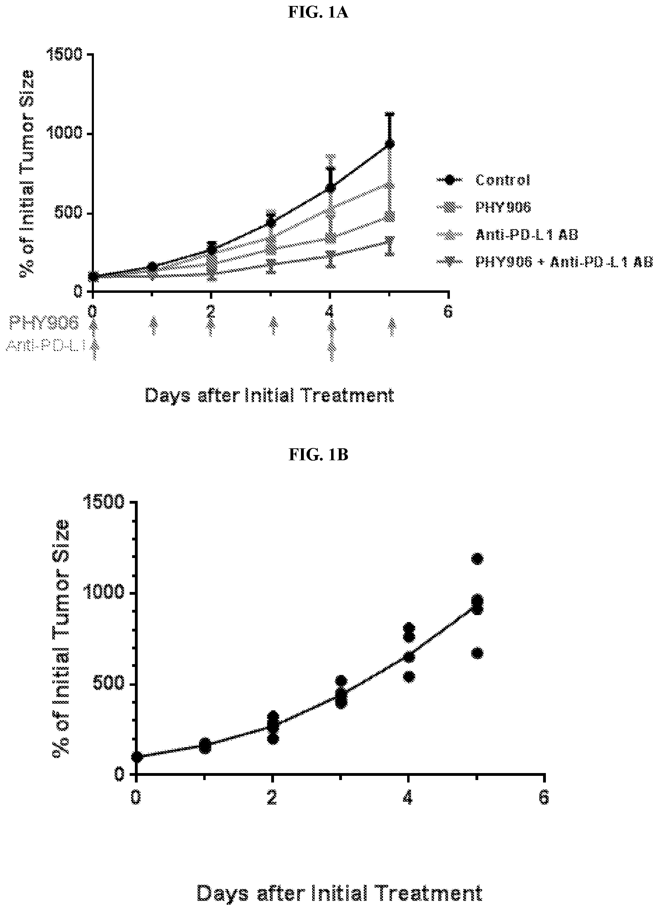

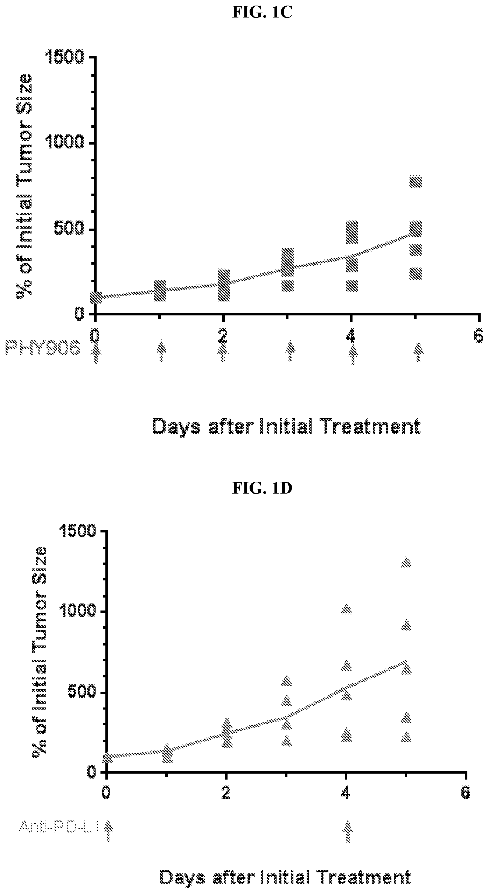

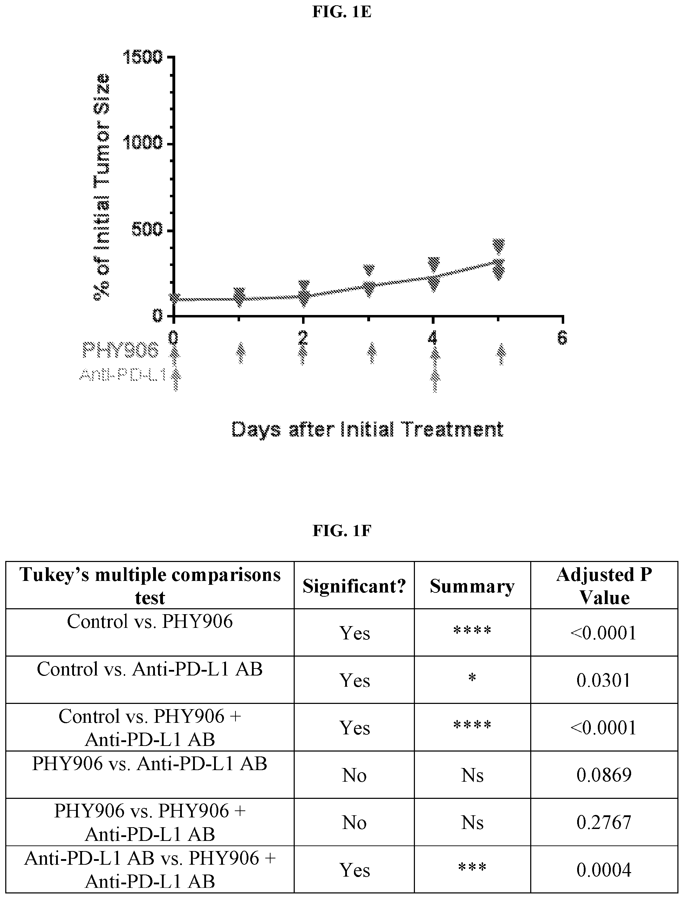

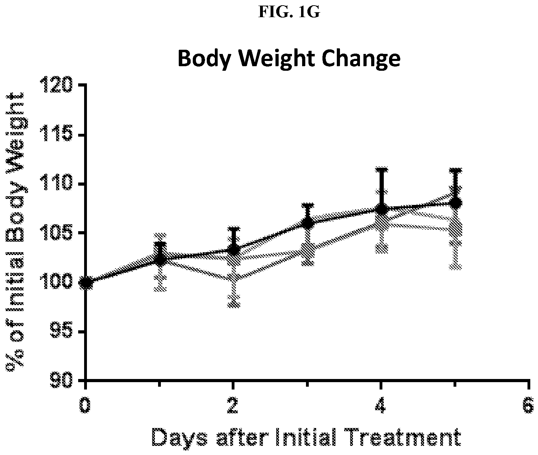

FIGs. 1A-1G depict graphs and a table reporting the effect of PHY906 and anti-PD-L1 on the growth of subcutaneously implanted B16 melanoma tumors in B6 mice. Administration was as follows: PHY906 (500 mg/kg, b.i.d., day 0 to day 5) and/or anti-PD-L1 (150 µg per animal, IP, day 0 and day 4). FIGs. 1A-1E illustrate relative tumor size following various treatment regimens. FIG. 1A illustrates a summary of four potential treatment regimens. FIG. 1B illustrates a control experiment. FIG. 1C illustrates treatment with only PHY906. FIG. 1D illustrates treatment with only anti-PD-L1. FIG. 1E illustrates treatment with both PHY906 and anti-PD-L1. FIG. 1F is a table illustrates significance of data reported in FIGs. 1A-1E, wherein P <0.05 indicated a significant result. FIG. 1G is a graph illustrating relative body weight change on each of the treatment regimens reported in FIGs. 1B-1F.

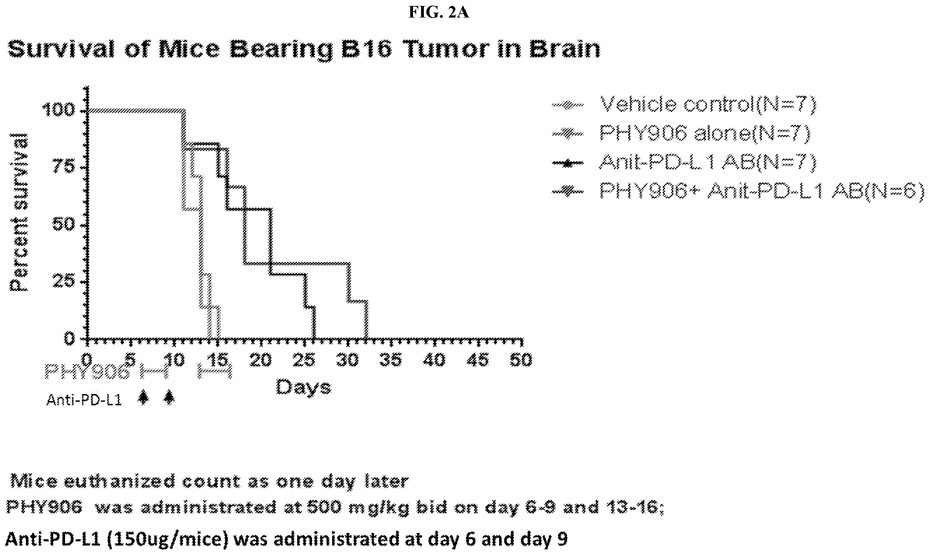

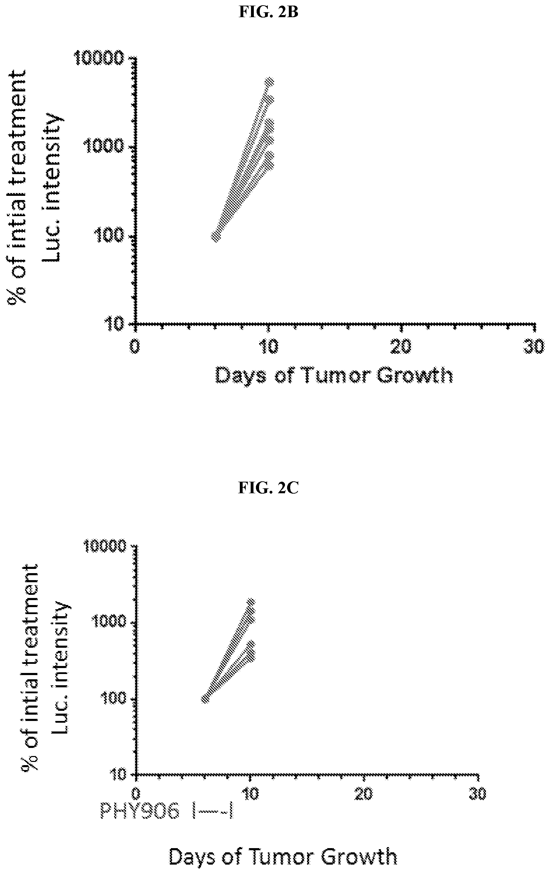

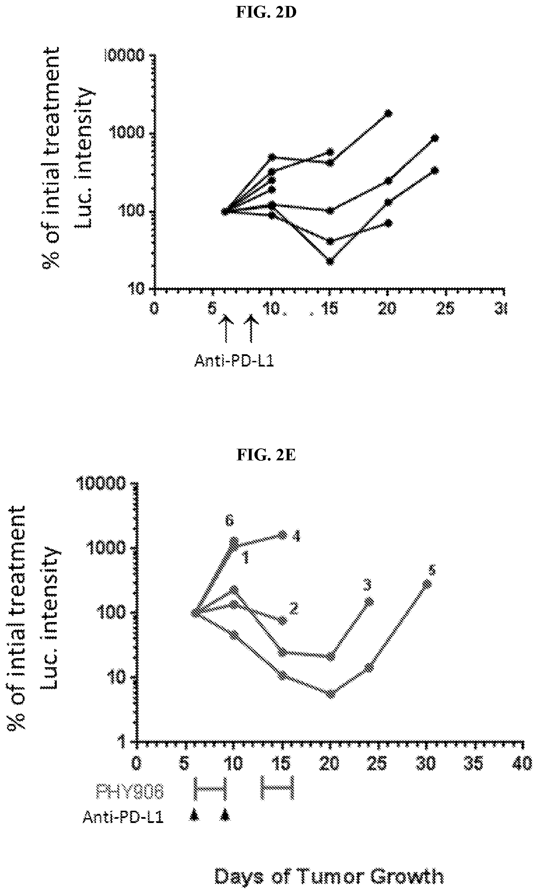

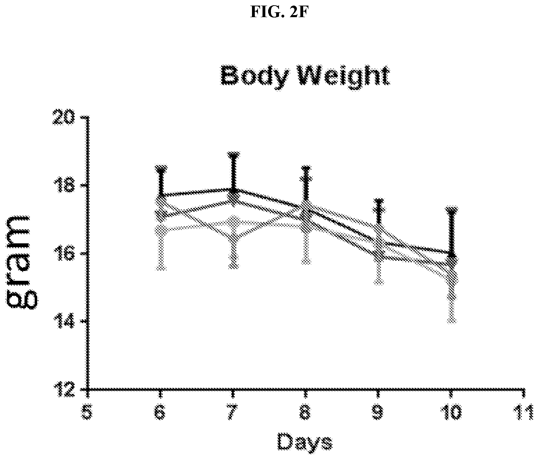

FIGs. 2A-2F depict graphs reporting the effect of PHY906 and anti-PD-L1 on the growth of brain implanted B16 melanoma tumors in B6 mice. FIG. 2A depicts a graph illustrating the survival rate of mice bearing B16 brain tumors following treatment with a control, PHY906 (500 mg/kg, b.i.d., on days 0 and 5), anti-PD-L1 (150 µg per animal, IP, on days 0 and 4) or both PHY906 and anti-PD-L1. FIG. 2B is a graph illustrating tumor luciferase intensity (as measured using luciferase imaging) after treatment with a control. FIG. 2C is a graph illustrating tumor luciferase intensity (as measured using luciferase imaging) after treatment with PHY906. FIG. 2D is a graph illustrating tumor luciferase intensity (as measured using luciferase imaging) after treatment with anti-PD-L1. FIG. 2E is a graph illustrating tumor luciferase intensity (as measured using luciferase imaging) after treatment with PHY906 and anti-PD-L1. FIG. 2F depicts a graph illustrating body weight (gram) change on each of the treatment regimens reported in FIGs. 2B-2E.

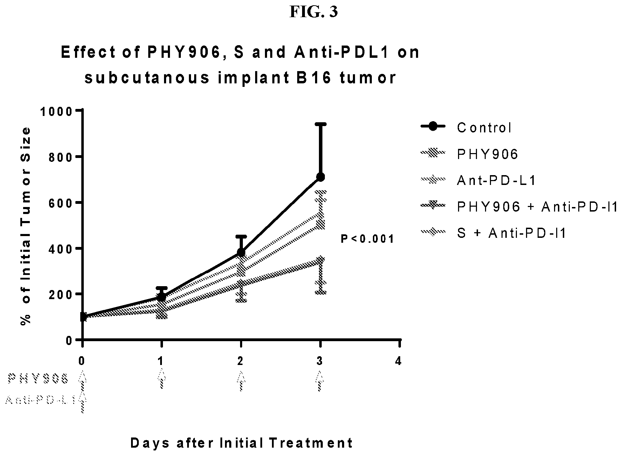

FIG. 3 depicts a graph illustrating the effects of PHY906 (500 mg/kg, b.i.d., on days 0, 1, 2 and 3), anti-PD-L1 (150 µg per animal, IP, on day 0), PHY906 + anti-PD-L1 and *Scutellaria baicalensis* (S, 500 mg/kg, b.i.d., on days 0, 1, 2 and 3) + anti-PD-L1 on the growth of subcutaneously implanted B16 melanoma tumors in B6 mice.
FIG. 4 is a table illustrating gastro-intestinal side effects of approved auto-immuno checkpoint therapy agents (adapted from Larkin, et al., 2005, N. Engl. Med. 373:23-34).

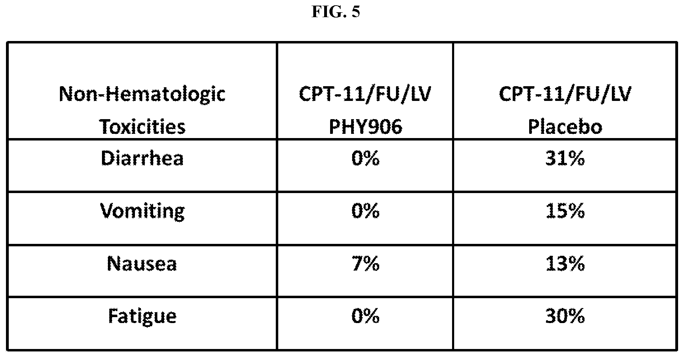

FIG. 5 is a table illustrating non-hematologic toxicities for irinotecan-based IFL chemotherapy in patients with advanced colorectal cancer (adapted from Kummar, et al., 2011, Clin. Colorectal Cancer 10(2):85-96).

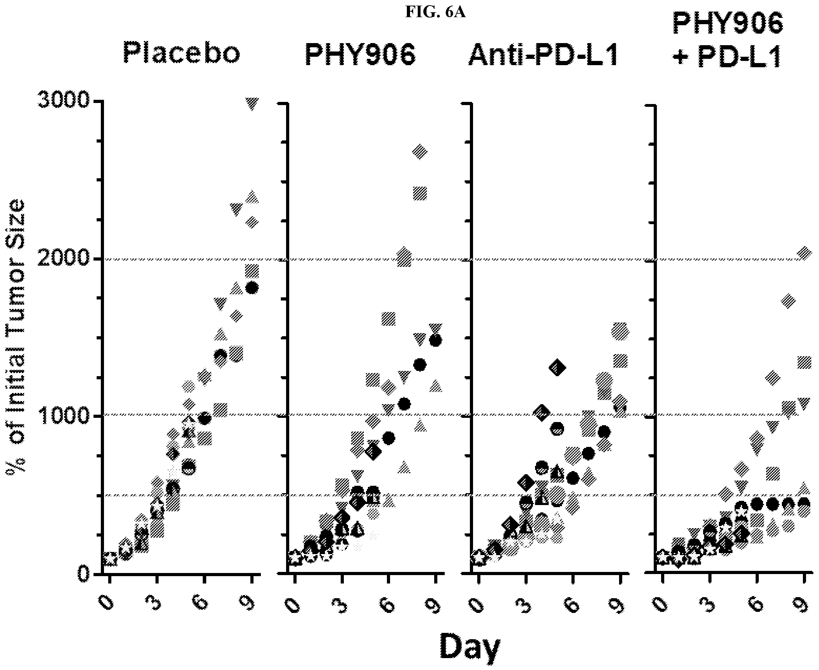

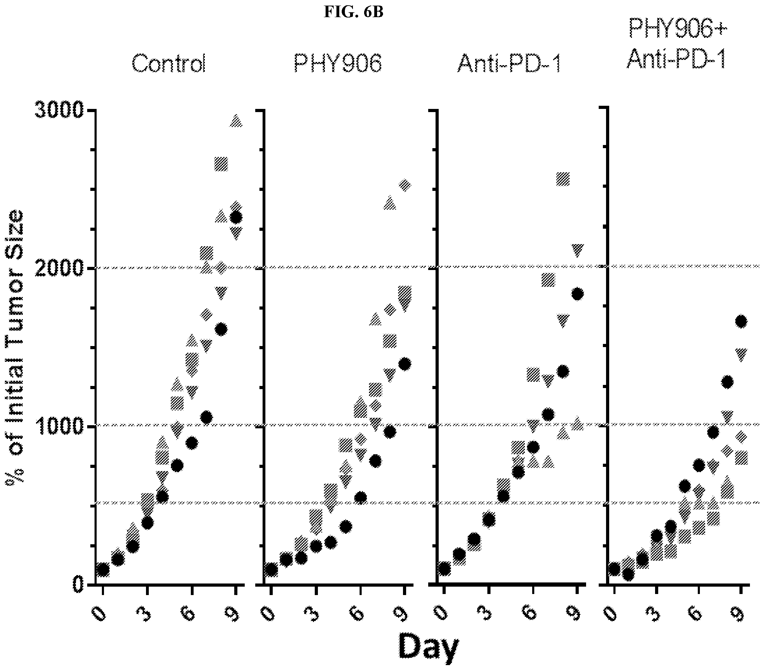

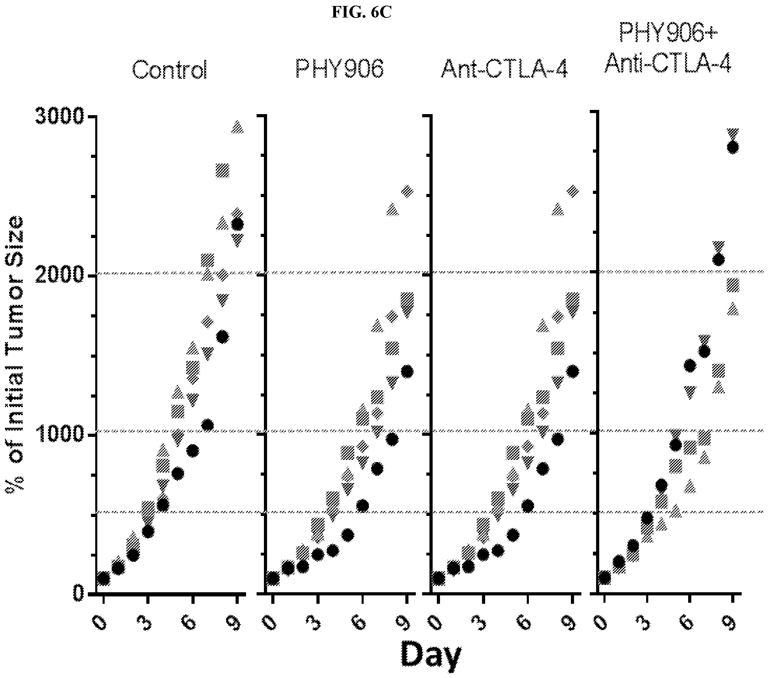

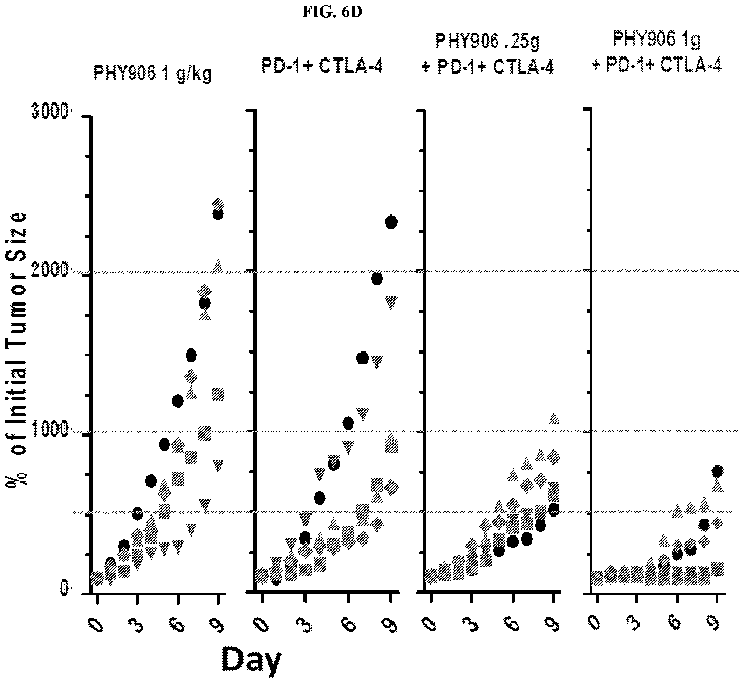

FIGs. 6A-6D are graphs reporting the effects of PHY906 alone and in combination with Anti-PD-L1, Anti-PD1 and Anti-CTLA4 antibodies against B16 melanoma in vivo. In FIGs. 6A-6C, PHY906 was administered in a dosage of 500 mg/kg twice per day, and where appropriate, Anti-PD-L1 (FIG. 6A, 150µg / animal), Anti-PD1 (FIG. 6B, 150µg / animal), and Anti-CTLA4 (FIG. 6C, 300µg / animal) were administered once every 3 days. In FIG. 6D, PHY906 was administered in dosages of 500 mg/kg or 1 g/kg twice per day as noted and Anti-PD1 (150µg / animal), and Anti-CTLA4 (300µg / animal) were administered once every 3 days.

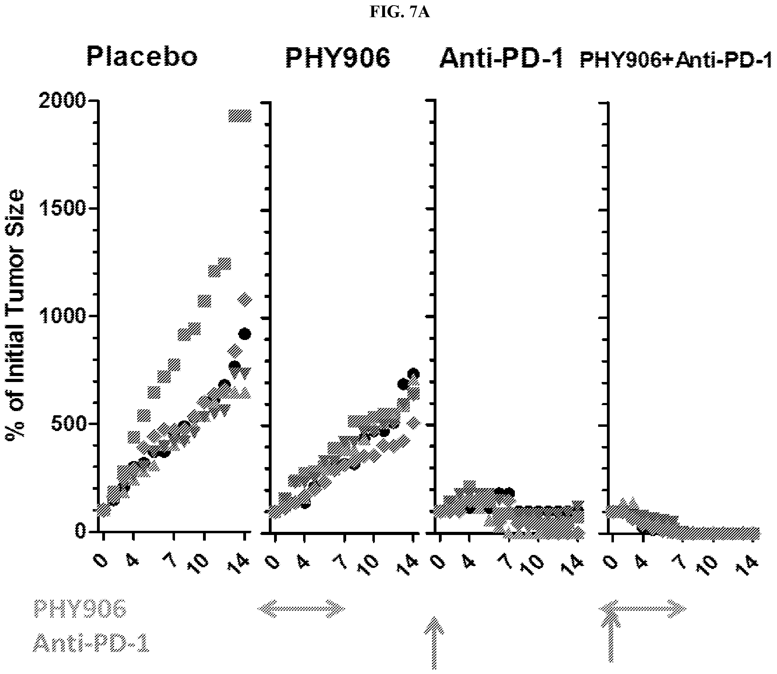

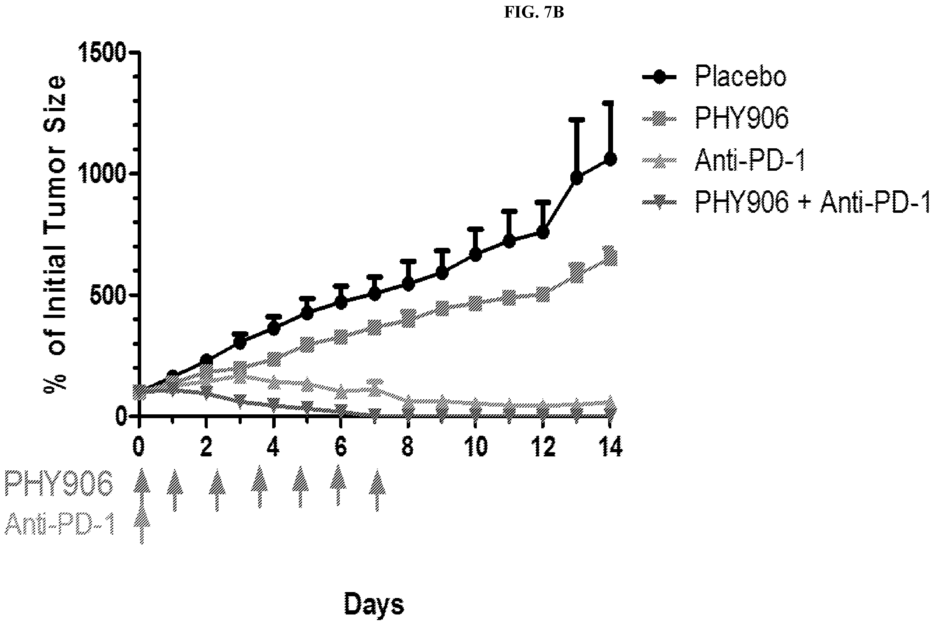

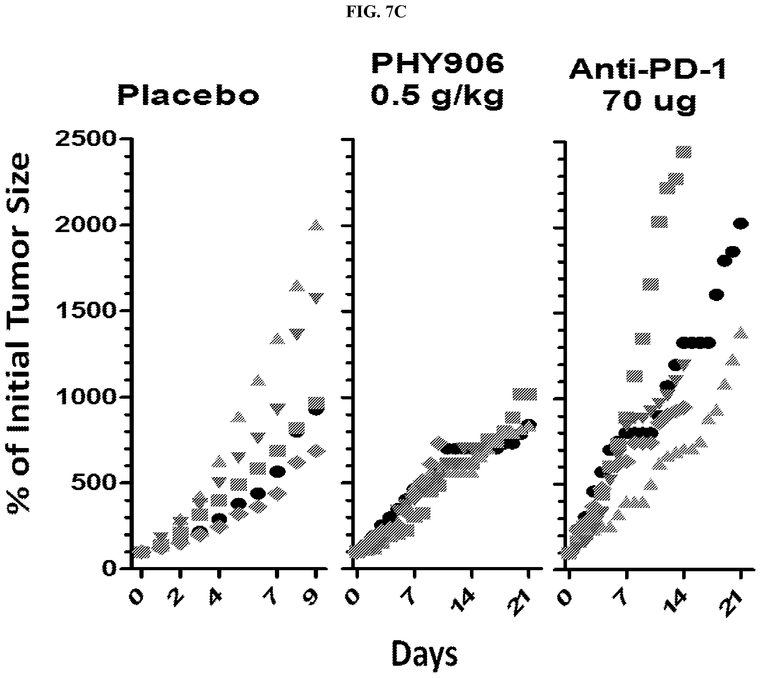

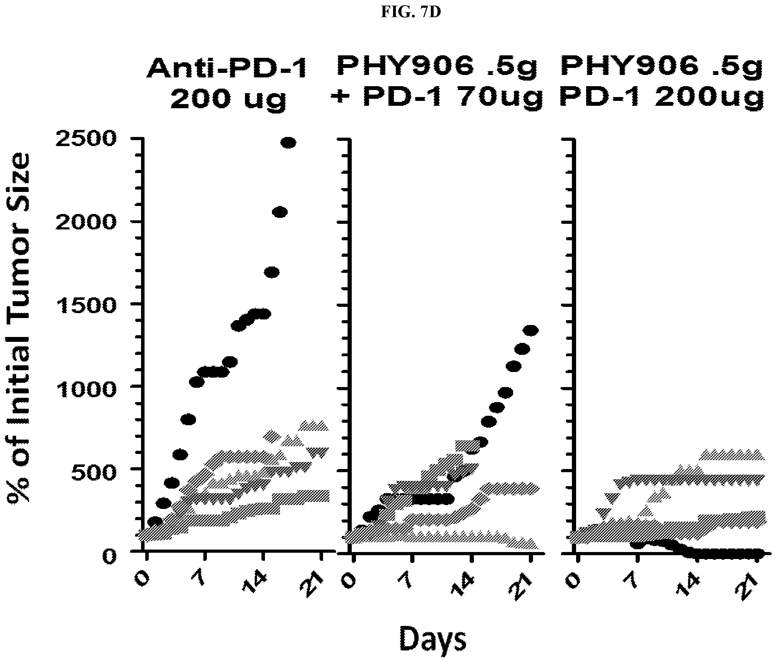

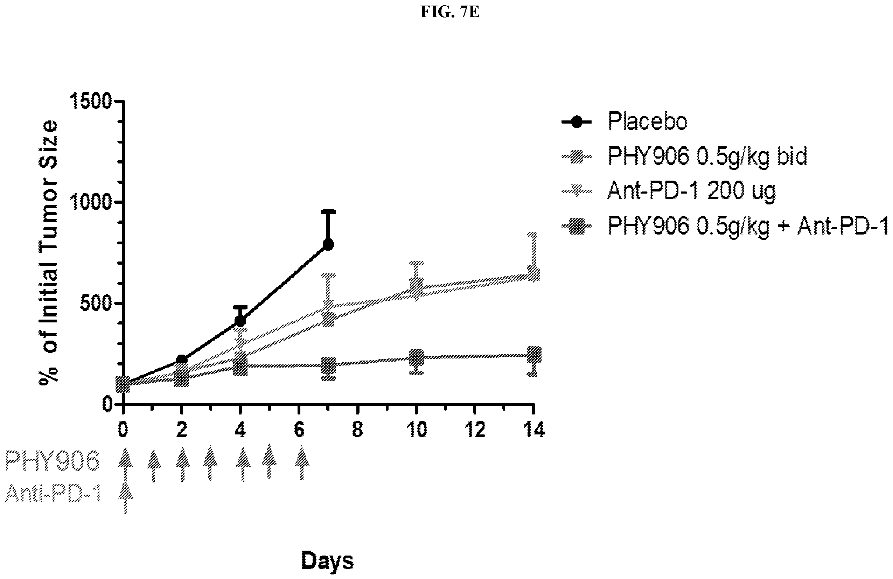

FIGs. 7A-7E are graphs reporting the effect of PHY906 alone and in combination with Anti-PD1 antibody on the size of Hepa 1-6 Mice hepatic adenocarcinoma tumors. In FIG. 7A, PHY906 was administered in a dosage of 500 mg/kg twice per day for 7 days. Anti-PD1 antibody was administered in a dosage of 200 µg / mouse once at the beginning of the experiment. FIG. 7B shows the change in tumor size averaged over multiple trials for each treatment regimen in FIG. 7A. FIGs. 7C and 7D are graphs reporting similar experiments to FIG. 7A, wherein PHY906 was administered in a dosage of 500 mg/kg twice per day for 7 days and Anti-PD1 antibody was administered in a dosage of 70 µg / mouse or 200 µg / mouse once at the beginning of the experiment. FIG. 7E shows the change in tumor size averaged over multiple trials for each treatment regimen in FIGs. 7C-7D.

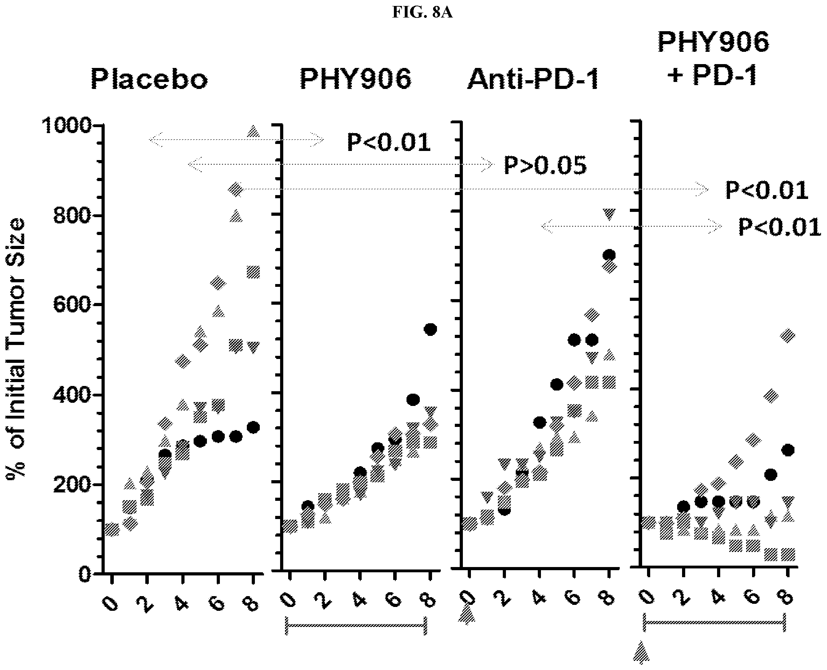

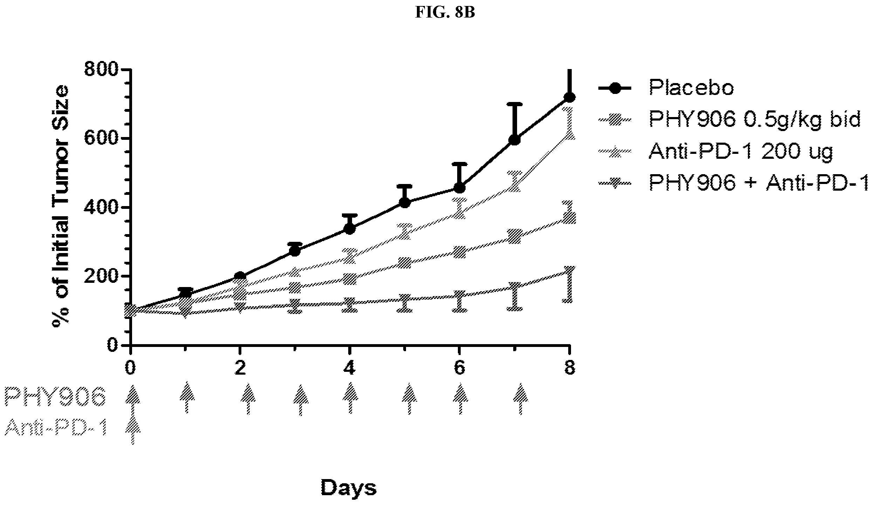

FIGs. 8A and 8B are graphs reporting the effect of PHY906 alone and in combination with Anti-PD1 antibody on the size of CMT-167 non-small cell lung cancer tumors. In FIG. 8A, PHY906 was administered in a dosage of 500 mg/kg twice per day for 7 days and Anti-PD1 antibody was administered in a dosage of 200 µg / mouse once at the beginning of the experiment. FIG. 8B shows the change in tumor size averaged over multiple trials for each treatment regimen in FIG. 8A.

### DETAILED DESCRIPTION OF THE INVENTION

The invention relates in one aspects to the unexpected discovery that any of the following compositions, or any combinations thereof, can be used to treat melanoma and other cancers in combination with one or more immune checkpoint inhibitors: (a) an herbal extract of *Scutellaria baicalensis* (S); (b) the herbal extract PHY906, which comprises herbal extracts of *Scutellaria baicalensis (S), Glycyrrhiza uralensis (G), Paeonia lactiflora* (P), and *Ziziphus jujuba* (Z) in a 3 : 2 : 2 : 2 ratio of S, G, P and Z; (c) an herbal extract comprising an extract of S; and (d) an herbal extract comprising a combination of S, G, P and Z. In certain embodiments, these herbal extracts, or isolated fractions thereof or active chemicals present therein, can be administered to a subject suffering from cancer concurrently or in combination with immune checkpoint inhibitors. In certain embodiments, the herbal extract comprises an aqueous extract, an alcohol extract, an acid extract, a base extract or any combinations thereof.

The invention also relates to kits comprising a pharmaceutical composition comprising one or more immune checkpoint inhibitors and any of the following compositions, or any combinations thereof: (a) an herbal extract of *Scutellaria baicalensis* (S); (b) the herbal extract PHY906, which comprises herbal extracts of *Scutellaria baicalensis (S), Glycyrrhiza uralensis (G), Paeonia lactiflora* (P), and *Ziziphus jujuba (Z)* in a 3 : 2 : 2 : 2 ratio of S, G, P and Z; (c) an herbal extract comprising an extract of S; and (d) an herbal extract comprising a combination of S, G, P and Z.

### Definitions

As used herein, each of the following terms has the meaning associated with it in this section.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, exemplary methods and materials are described.

Generally, the nomenclature used herein and the laboratory procedures in pharmacology, natural product chemistry, and organic chemistry are those well-known and commonly employed in the art.

As used herein, the articles "a" and "an" refer to one or to more than one *(i.e.,* to at least one) of the grammatical object of the article. By way of example, "an element" means one element or more than one element.

As used herein, the term "cancer" is defined as disease characterized by the rapid and uncontrolled growth of aberrant cells. Cancer cells can spread locally or through the bloodstream and lymphatic system to other parts of the body. Examples of various cancers include but are not limited to, bone cancer, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

In one aspect, the terms "co-administered" and "co-administration" as relating to a subject refer to administering to the subject a compound and/or composition of the invention along with a compound and/or composition that may also treat or prevent a disease or disorder contemplated herein. In certain embodiments, the co-administered compounds and/or compositions are administered separately, or in any kind of combination as part of a single therapeutic approach. The co-administered compound and/or composition may be formulated in any kind of combinations as mixtures of solids and liquids under a variety of solid, gel, and liquid formulations, and as a solution.

As used herein, the term "extract" refers to a concentrated preparation or solution of a compound or drug derived from a naturally occurring source, such as an herb or other plant material. Extracts may be prepared by a number of processes, including steeping an herb in solution, or drying and grinding an herb into a powder and dissolving the powder in a solution. An extract may be further concentrated by removing a portion of the solvent after dissolving an amount of the desired compound in the solution. An extract may also be strained or centrifuged to remove any solid material from the solution.

The phrase "inhibit," as used herein, means to reduce a molecule, a reaction, an interaction, a gene and/or a protein's expression, stability, function or activity by a measurable amount or to prevent entirely. Inhibitors are compounds that, *e.g.,* bind to, partially or totally block stimulation, decrease, prevent, delay activation, inactivate, desensitize, or down regulate a protein or a gene's stability, expression, function and activity, e.g., antagonists.

As used herein, the term "pharmaceutical composition" or "composition" refers to a mixture of at least one compound useful within the invention with a pharmaceutically acceptable carrier. The pharmaceutical composition facilitates administration of the compound to a subject.

As used herein, the term "pharmaceutically acceptable" refers to a material, such as a carrier or diluent, which does not abrogate the biological activity or properties of the compound useful within the invention, and is relatively non-toxic, *i.e.,* the material may be administered to a subject without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the subject such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the subject. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA).

As used herein, the language "pharmaceutically acceptable salt" refers to a salt of the administered compound prepared from pharmaceutically acceptable non-toxic acids and bases, including inorganic acids, inorganic bases, organic acids, inorganic bases, solvates, hydrates, and clathrates thereof. Suitable pharmaceutically acceptable acid addition salts may be prepared from an inorganic acid or from an organic acid. Examples of inorganic acids include sulfate, hydrogen sulfate, hydrochloric, hydrobromic, hydriodic, nitric, carbonic, sulfuric, and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid. Suitable pharmaceutically acceptable base addition salts of compounds of the invention include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylene-diamine, chloroprocaine, choline, diethanolamine, ethylenediamine, meglumine (N methylglucamine) and procaine. All of these salts may be prepared from the corresponding compound by reacting, for example, the appropriate acid or base with the compound.

The terms "pharmaceutically effective amount" and "effective amount" refer to a non-toxic but sufficient amount of an agent to provide the desired biological result. That result can be reduction and/or alleviation of the signs, symptoms, or causes of a disease or disorder, or any other desired alteration of a biological system. An appropriate effective amount in any individual case may be determined by one of ordinary skill in the art using routine experimentation. By "pharmaceutical formulation" it is further meant that the carrier, solvent, excipient(s) and/or salt must be compatible with the active ingredient of the formulation (e.g. a compound of the invention). It is understood by those of ordinary skill in this art that the terms "pharmaceutical formulation" and "pharmaceutical composition" are generally interchangeable, and they are so used for the purposes of this application.

As used herein, the term "PHY906" refers to an herbal composition comprising *Glycyrrhiza uralensis Fisch (G), Paeonia lactiflora Pall (P), Scutellaria baicalensis Georgi* (S), and *Ziziphus jujuba Mill (Z).* PHY906 refers to a specific composition comprising S, G, P and Z in a 3 : 2 : 2 : 2 ratio prepared under standard operational procedure.

As used herein, the term "prevent," "prevention," or "preventing" refers to any method to partially or completely prevent or delay the onset of one or more symptoms or features of a disease, disorder, and/or condition, for example, cancer. Prevention is causing the clinical symptoms of the disease state not to develop, *i.e*., inhibiting the onset of disease, in a subject that may be exposed to or predisposed to the disease state, but does not yet experience or display symptoms of the disease state. Prevention may be administered to a subject who does not exhibit signs of a disease, disorder, and/or condition.

As used herein, the term "subject," "patient" or "individual" to which administration is contemplated includes, but is not limited to, humans (*i.e*., a male or female of any age group, *e.g.,* a pediatric subject (e.g., infant, child, adolescent) or adult subject *(e.g.,* young adult, middle-aged adult or senior adult)) and/or other primates (*e.g*., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys.

As used herein, the term "therapeutically effective amount" is an amount of a compound of the invention, that when administered to a patient, treats, minimizes and/or ameliorates a symptom of the disease or disorder. The amount of a compound of the invention that constitutes a "therapeutically effective amount" will vary depending on the compound, the disease state and its severity, the age of the patient to be treated, and the like. The therapeutically effective amount can be determined routinely by one of ordinary skill in the art having regard to his own knowledge and to this disclosure.

As used herein, the term "treatment" or "treating" is defined as the application or administration of a therapeutic agent, *i.e.* , a compound useful within the invention (alone or in combination with another pharmaceutical agent), to a subject, or application or administration of a therapeutic agent to an isolated tissue or cell line from a subject ( *e.g.* , for diagnosis or *ex vivo* applications), who has cancer, a symptom of cancer or the potential to develop cancer, with the purpose to cure, heal, alleviate, relieve, alter, remedy, ameliorate, improve or affect cancer, the symptoms of cancer or the potential to develop cancer. Such treatments may be specifically tailored or modified, based on knowledge obtained from the field of pharmacogenomics.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual and partial numbers within that range, for example, 1, 2, 2.7, 3, 4, 5, 5.3, and 6. This applies regardless of the breadth of the range.

The following abbreviations are used herein:
- cGMP: current Good Manufacturing Practice
- G: *Glycyrrhiza uralensis Fisch,* also known as Chinese liquorice
- IFNγ: interferon-gamma
- P: *Paeonia lactiflora Pall,* also known as Chinese peony
- S: *Scutellaria baicalensis Georgi,* also known as Baikal skullcap or scute
- Z: *Ziziphus jujube Mill,* also known as red date or Chinese date

### Compositions

The present invention relates to compounds and compositions for the treatment of cancer. In certain embodiments, the compositions for the treatment of cancer are useful for treating melanoma and other cancers, reducing the gastro-intestinal toxicity caused by an anti-PD-L1, anti-PD1 or anti-CTLA4, while not affecting their anti-tumor activity.

The compositions for the treatment of cancer comprise an herbal extract of *Scutellaria baicalensis* (S).

The compositions for the treatment of cancer comprise the herbal extract PHY906, which comprises herbal extracts of *Scutellaria baicalensis (S), Glycyrrhiza uralensis (G), Paeonia lactiflora* (P), and *Ziziphus jujuba (Z)* in a 3 : 2 : 2 : 2 ratio of S, G, P and Z.

The compositions for the treatment of cancer comprise an herbal extract comprising an extract of S.

The compositions for the treatment of cancer comprise an herbal extract comprising a combination of S, G, P and Z.

In certain embodiments, the compositions for the treatment of cancer further comprise an herbal extract comprising a combination of G, P and Z.

In certain embodiments, the compositions comprise one or more immune checkpoint inhibitors.

In certain embodiments, the compositions further comprise one or more pharmaceutically acceptable carriers.

In certain embodiments, the one or more immune checkpoint inhibitors are selected from the group consisting of an anti-PD1, an anti-PD-L1 and an anti-CTLA4. In other embodiments, the immune checkpoint inhibitor is selected from the group consisting of Ipilimumab (YERVOY^{®}), which targets CTLA4, Pembrolizumab (KEYTRUDA^{®}) and Nivolumab (OPDIVO^{®}), which target PD1, and Durvalumab and Atezolizumab (TECENTRIQ^{®}), which target PD-L1.

### Uses in methods of treatment

The present invention includes using the compositions for the treatment of cancer described herein above in methods of treating cancer in a subject, the method comprising administering to the subject in need thereof a therapeutically effective amount of a composition for the treatment of cancer as disclosed herein. The method comprises administering to the subject at least one selected from the group consisting of: (a) an herbal extract of *Scutellaria baicalensis* (S); (b) the herbal extract PHY906, which comprises herbal extracts of *Scutellaria baicalensis (S), Glycyrrhiza uralensis (G), Paeonia lactiflora* (P), and *Ziziphus jujuba* (Z) in a 3 : 2 : 2: 2 ratio of S, G, P and Z; (c) an herbal extract comprising an extract of S; and (d) an herbal extract comprising a combination of S, G, P and Z; and coadministering one or more immune checkpoint inhibitors to the subject

selected from the group consisting of an anti-PD1, an anti-PD-L1 and an anti-CTLA4. In certain embodiments, the immune checkpoint inhibitor is selected from the group consisting of Ipilimumab (YERVOY^{®}), which targets CTLA4, Pembrolizumab (KEYTRUDA^{®}) and Nivolumab (OPDIVO^{®}), which target PD1, and Durvalumab and Atezolizumab (TECENTRIQ^{®}), which target PD-L1.

In certain embodiments, the one or more herbal extracts are administered to the subject orally. In other embodiments, the one or more herbal extracts are administered in one or more forms selected from the group consisting of a pill, tablet, capsule, soup, tea, concentrate, dragees, liquids, drops, and gelcaps.

In certain embodiments the therapeutically effective amount of the one or more herbal extracts is 20 mg/day to 2 g/day, in certain embodiments 100 mg/day to 2 g/day, and in other embodiments 1.6 g/day.

In certain embodiments, the herbal extracts of the invention can be administered twice per day, in doses of 800 mg *(i.e.,* 800 mg, twice per day or 1,600 mg/day). In certain embodiments, the herbal extracts are administered once or twice per day for 1 to 2 weeks. In certain embodiments, the herbal extracts are administered for 1 to 2 weeks, followed by a suspension of treatment for one or more weeks.

In certain embodiments, the therapeutically effective amount of the herbal extracts counteracts one or more gastrointestinal side effects of the immune checkpoint inhibitors. In certain non-limiting embodiments, the side effects are at least one selected from the group consisting of nausea, vomiting, fatigue, colitis and diarrhea.

In certain embodiments, the cancer is at least one selected from the group consisting of, but not necessarily limited to, melanoma, non-small cell lung cancer, renal cell carcinoma, liver cancer, colon cancer, urothelial bladder cancer, and pancreatic cancer.

In certain embodiments, the herbal extracts and the one or more immune checkpoint inhibitors exhibit a synergistic effect. In certain embodiments, the therapeutically effective amount of the immune checkpoint inhibitor is lower when administered as part of the methods of the invention, as compared to when it is administered alone. In other embodiments, the therapeutically effective course of treatment with the immune checkpoint inhibitor is shorter when administered as part of the methods of the invention, as compared to when it is administered alone. In yet other embodiments, the tumor shrinking and/or tumor inhibiting effect of the immune checkpoint inhibitor is greater when administered as part of the methods of the invention, as compared to when it is administered alone.

In certain embodiments, the subject is a mammal. In other embodiments, the subject is human.

### Administration/Dosage/Formulations

The regimen of administration may affect what constitutes an effective amount. The therapeutic formulations may be administered to the subject either prior to or after the onset of a disease or disorder contemplated in the invention. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the compositions of the present invention to a patient, preferably a mammal, more preferably a human, may be carried out using known procedures, at dosages and for periods of time effective to treat a disease or disorder contemplated in the invention. An effective amount of the therapeutic compound necessary to achieve a therapeutic effect may vary according to factors such as the state of the disease or disorder in the patient; the age, sex, and weight of the patient; and the ability of the therapeutic compound to treat a disease or disorder contemplated in the invention. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention is from about 1 and 1,000 mg/kg of body weight/per day. The pharmaceutical compositions useful for practicing the invention may be administered to deliver a dose of from 1 ng/kg/day and 100 mg/kg/day. One of ordinary skill in the art would be able to study the relevant factors and make the determination regarding the effective amount of the therapeutic compound without undue experimentation.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

In particular, the selected dosage level depends upon a variety of factors including the activity of the particular compound employed, the time of administration, the rate of excretion of the compound, the duration of the treatment, other drugs, compounds or materials used in combination with the compound, the age, sex, weight, condition, general health and prior medical history of the patient being treated, and like factors well known in the medical arts.

A medical doctor, e.g., physician or veterinarian, having ordinary skill in the art may readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In particular embodiments, it is advantageous to formulate the compound in dosage unit form for ease of administration and uniformity of dosage. Dosage unit form as used herein refers to physically discrete units suited as unitary dosages for the patients to be treated; each unit containing a predetermined quantity of therapeutic compound calculated to produce the desired therapeutic effect in association with the required pharmaceutical vehicle. The dosage unit forms of the invention are dictated by and directly dependent on (a) the unique characteristics of the therapeutic compound and the particular therapeutic effect to be achieved, and (b) the limitations inherent in the art of compounding/formulating such a therapeutic compound for the treatment of a disease or disorder contemplated in the invention.

In certain embodiments, the compositions of the invention are formulated using one or more pharmaceutically acceptable excipients or carriers. In other embodiments, the pharmaceutical compositions of the invention comprise a therapeutically effective amount of a compound of the invention and a pharmaceutically acceptable carrier. In yet other embodiments, the compound of the invention is the only biologically active agent *(i.e.,* capable of treating cancer) in the composition. In yet other embodiments, the compound of the invention is the only biologically active agent *(i.e.,* capable of treating cancer) in therapeutically effective amounts in the composition.

The carrier may be a solvent or dispersion medium containing, for example, water, ethanol, polyol (for example, glycerol, propylene glycol, and liquid polyethylene glycol, and the like), suitable mixtures thereof, and vegetable oils. The proper fluidity may be maintained, for example, by the use of a coating such as lecithin, by the maintenance of the required particle size in the case of dispersion and by the use of surfactants. Prevention of the action of microorganisms may be achieved by various antibacterial and antifungal agents, for example, parabens, chlorobutanol, phenol, ascorbic acid, thimerosal, and the like. In many cases, it is preferable to include isotonic agents, for example, sugars, sodium chloride, or polyalcohols such as mannitol and sorbitol, in the composition. Prolonged absorption of the injectable compositions may be brought about by including in the composition an agent which delays absorption, for example, aluminum monostearate or gelatin.

In certain embodiments, the compositions of the invention are administered to the patient in dosages that range from one to five times per day or more. **In** other embodiments, the compositions of the invention are administered to the patient in range of dosages that include, but are not limited to, once every day, every two days, every three days to once a week, and once every two weeks. It is readily apparent to one skilled in the art that the frequency of administration of the various combination compositions of the invention varies from individual to individual depending on many factors including, but not limited to, age, disease or disorder to be treated, gender, overall health, and other factors. Thus, the invention should not be construed to be limited to any particular dosage regime and the precise dosage and composition to be administered to any patient is determined by the attending physical taking all other factors about the patient into account.

Compounds and/or compositions of the invention for administration may be in the range of from about 1 mg to about 10,000 mg, about 20 mg to about 9,500 mg, about 40 mg to about 9,000 mg, about 75 mg to about 8,500 mg, about 150 mg to about 7,500 mg, about 200 mg to about 7,000 mg, about 400 mg to about 6,000 mg, about 500 mg to about 5,000 mg, about 750 mg to about 4,000 mg, about 1,000 mg to about 3,000 mg, about 1,000 mg to about 2,500 mg, about 20 mg to about 2,000 mg and any and all whole or partial increments therebetween. In certain embodiments, the dose of the compounds and/or compositions of the invention is about 800 mg.

In certain embodiments, the present invention is directed to a packaged pharmaceutical composition comprising a container holding a therapeutically effective amount of a compound of the invention, alone or in combination with a second pharmaceutical agent; and instructions for using the compound to treat, prevent, or reduce one or more symptoms of a disease or disorder contemplated in the invention.

Formulations may be employed in admixtures with conventional excipients, *i.e.,* pharmaceutically acceptable organic or inorganic carrier substances suitable for oral, parenteral, nasal, intravenous, subcutaneous, enteral, or any other suitable mode of administration, known to the art. The pharmaceutical preparations may be sterilized and if desired mixed with auxiliary agents, e.g., lubricants, preservatives, stabilizers, wetting agents, emulsifiers, salts for influencing osmotic pressure buffers, coloring, flavoring and/or aromatic substances and the like. They may also be combined where desired with other active agents.

Routes of administration of any of the compositions of the invention include oral nasal, rectal, intravaginal, parenteral, buccal, sublingual or topical. The compounds for use in the invention may be formulated for administration by any suitable route, such as for oral or parenteral, for example, transdermal, transmucosal (*e.g*., sublingual, lingual, (trans)buccal, (trans)urethral, vaginal (*e.g*., trans- and perivaginally), (intra)nasal and (trans)rectal), intravesical, intrapulmonary, intraduodenal, intragastrical, intrathecal, subcutaneous, intramuscular, intradermal, intra-peritoneal, intra-arterial, intravenous, intrabronchial, inhalation, and topical administration.

Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein.

### Oral Administration

For oral application, particularly suitable are soups, teas, concentrates, tablets, dragees, liquids, drops, suppositories, or capsules, caplets and gelcaps. The compositions intended for oral use may be prepared according to any method known in the art and such compositions may contain one or more agents selected from the group consisting of inert, non-toxic pharmaceutically excipients that are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay the release of the active ingredients. Formulations for oral use may also be presented as hard gelatin capsules wherein the active ingredient is mixed with an inert diluent.

For oral administration, the compounds of the invention may be in the form of tablets or capsules prepared by conventional means with pharmaceutically acceptable excipients such as binding agents (*e.g*., polyvinylpyrrolidone, hydroxypropylcellulose or hydroxypropylmethylcellulose); fillers (*e.g*., cornstarch, lactose, microcrystalline cellulose or calcium phosphate); lubricants *(e.g.,* magnesium stearate, talc, or silica); disintegrates *(e.g.,* sodium starch glycollate); or wetting agents (*e.g*., sodium lauryl sulphate). If desired, the tablets may be coated using suitable methods and coating materials such as OPADRY^{™} film coating systems available from Colorcon, West Point, Pa. (*e.g*., OPADRY^{™} OY Type, OYC Type, Organic Enteric OY-P Type, Aqueous Enteric OY-A Type, OY-PM Type and OPADRY^{™} White, 32K18400). Liquid preparation for oral administration may be in the form of solutions, syrups or suspensions. The liquid preparations may be prepared by conventional means with pharmaceutically acceptable additives such as suspending agents *(e.g.,* sorbitol syrup, methyl cellulose or hydrogenated edible fats); emulsifying agent *(e.g.,* lecithin or acacia); non-aqueous vehicles (*e.g*., almond oil, oily esters or ethyl alcohol); and preservatives (*e.g*., methyl or propyl p-hydroxy benzoates or sorbic acid).

Granulating techniques are well known in the pharmaceutical art for modifying starting powders or other particulate materials of an active ingredient. The powders are typically mixed with a binder material into larger permanent free-flowing agglomerates or granules referred to as a "granulation". For example, solvent-using "wet" granulation processes are generally characterized in that the powders are combined with a binder material and moistened with water or an organic solvent under conditions resulting in the formation of a wet granulated mass from which the solvent must then be evaporated.

Melt granulation generally consists in the use of materials that are solid or semi-solid at room temperature *(i.e.,* having a relatively low softening or melting point range) to promote granulation of powdered or other materials, essentially in the absence of added water or other liquid solvents. The low melting solids, when heated to a temperature in the melting point range, liquefy to act as a binder or granulating medium. The liquefied solid spreads itself over the surface of powdered materials with which it is contacted, and on cooling, forms a solid granulated mass in which the initial materials are bound together. The resulting melt granulation may then be provided to a tablet press or be encapsulated for preparing the oral dosage form. Melt granulation improves the dissolution rate and bioavailability of an active *(i.e.,* drug) by forming a solid dispersion or solid solution.

U.S. Patent No. 5,169,645 discloses directly compressible wax-containing granules having improved flow properties. The granules are obtained when waxes are admixed in the melt with certain flow improving additives, followed by cooling and granulation of the admixture. In certain embodiments, only the wax itself melts in the melt combination of the wax(es) and additives(s), and in other cases both the wax(es) and the additives(s) melt.

The present invention also includes a multi-layer tablet comprising a layer providing for the delayed release of one or more compounds of the invention, and a further layer providing for the immediate release of a medication for treatment of a disease or disorder contemplated in the invention. Using a wax/pH-sensitive polymer mix, a gastric insoluble composition may be obtained in which the active ingredient is entrapped, ensuring its delayed release.

### Parenteral Administration

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intra-peritoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multidose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry *(i.e.,* powder or granular) form for reconstitution with a suitable vehicle (*e.g*., sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### Controlled Release Formulations and Drug Delivery Systems

In certain embodiments, the formulations of the present invention may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release which is longer that the same amount of agent administered in bolus form.

For sustained release, the compounds may be formulated with a suitable polymer or hydrophobic material that provides sustained release properties to the compounds. As such, the compounds useful within the methods of the invention may be administered in the form of microparticles, for example by injection, or in the form of wafers or discs by implantation.

In one embodiment of the invention, the compounds of the invention are administered to a patient, alone or in combination with another pharmaceutical agent, using a sustained release formulation.

The term delayed release is used herein in its conventional sense to refer to a drug formulation that provides for an initial release of the drug after some delay following drug administration and that may, although not necessarily, includes a delay of from about 10 minutes up to about 12 hours.

The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration.

As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, about 10 minutes, or about 1 minute and any or all whole or partial increments thereof after drug administration after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, about 10 minutes, or about 1 minute and any and all whole or partial increments thereof after drug administration.

### Dosing

The therapeutically effective amount or dose of a compound of the present invention depends on the age and weight of the patient, the current medical condition of the patient and the progression of a disease or disorder contemplated in the invention. The skilled artisan is able to determine appropriate dosages depending on these and other factors.

A suitable dose of a compound of the present invention may be in the range of from about 0.01 mg to about 5,000 mg per day, such as from about 0.1 mg to about 1,000 mg, for example, from about 1 mg to about 500 mg, such as about 5 mg to about 250 mg per day. The dose may be administered in a single dosage or in multiple dosages, for example from 1 to 5 or more times per day. When multiple dosages are used, the amount of each dosage may be the same or different. For example, a dose of 1 mg per day may be administered as two 0.5 mg doses, with about a 12-hour interval between doses.

It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on.

In the case wherein the patient's status does improve, upon the doctor's discretion the administration of the inhibitor of the invention is optionally given continuously; alternatively, the dose of drug being administered is temporarily reduced or temporarily suspended for a certain length of time *(i.e.,* a "drug holiday"). The length of the drug holiday optionally varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday includes from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, is reduced, as a function of the disease or disorder, to a level at which the improved disease is retained. In certain embodiments, patients require intermittent treatment on a long-term basis upon any recurrence of symptoms and/or infection.

The compounds for use in the method of the invention may be formulated in unit dosage form. The term "unit dosage form" refers to physically discrete units suitable as unitary dosage for patients undergoing treatment, with each unit containing a predetermined quantity of active material calculated to produce the desired therapeutic effect, optionally in association with a suitable pharmaceutical carrier. The unit dosage form may be for a single daily dose or one of multiple daily doses (*e.g*., about 1 to 5 or more times per day). When multiple daily doses are used, the unit dosage form may be the same or different for each dose.

Toxicity and therapeutic efficacy of such therapeutic regimens are optionally determined in experimental animals, including, but not limited to, the determination of the LD₅₀ (the dose lethal to 50% of the population) and the ED₅₀ (the dose therapeutically effective in 50% of the population). The dose ratio between the toxic and therapeutic effects is the therapeutic index, which is expressed as the ratio between LD₅₀ and ED₅₀. The data obtained from animal studies are optionally used in formulating a range of dosage for use in human. The dosage of such compounds lies preferably within a range of circulating concentrations that include the ED₅₀ with minimal toxicity. The dosage optionally varies within this range depending upon the dosage form employed and the route of administration utilized.

Those skilled in the art will recognize, or be able to ascertain using no more than routine experimentation, numerous equivalents to the specific procedures, embodiments, claims, and examples described herein.

For example, it should be understood, that modifications in reaction conditions, including but not limited to reaction times, reaction size/volume, and experimental reagents with art-recognized alternatives and using no more than routine experimentation, are within the scope of the present

### invention as defined by the appended claims.

It is to be understood that, wherever values and ranges are provided herein, the description in range format is merely for convenience and brevity and should not be construed as an inflexible limitation on the scope of the invention. Accordingly, all values and ranges encompassed by these values and ranges are meant to be encompassed within the scope of the present invention. Moreover, all values that fall within these ranges, as well as the upper or lower limits of a range of values, are also contemplated by the present application. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present invention. However, they are in no way a limitation of the teachings or disclosure of the present invention as set forth in the appended claims.

### EXAMPLES

The invention is now described with reference to the following Examples. These Examples are provided for the purpose of illustration only and the invention should in no way be construed as being limited to these Examples.

### Materials and Methods

B16 B10 luciferase mouse melanoma cells were used for tumor implementation (subcutaneously or brain implementation) in C57BL6 mice. Mouse monoclonal anti-PD-L1 (clone 10B5) (150 µg/mouse) was used in all the experiment. PHY906 (500mg/kg, b.i.d.) water extract was used in the experiments.

B16 B10 luciferase mouse melanoma cells were implanted subcutaneously (10⁶ cells per mice) or in the brain (10³ cell per mice) in C57BL6 mice. Treatment was started after 6 days of tumor implantation. Mouse monoclonal anti-PD-L1 (clone 10B5) (150 µg/mouse every 3 days) was used in all the experiment. PHY906 water extract were fed orally to mice 500mg/kg, b.i.d.: daily for subcutaneously implemented B16 tumor; from day 6-9 and day 13-16 for brain B16 tumor. Mice with tumor sizes of 150-300mm³ were selected. Tumor volume was estimated by using the formula (length x width²)/2. For luciferase imaging, luciferin 25mg/kg was injected to each mice and luciferase imaging was recorded using IVIS imaging system.

### Example 1: Preparation of herbal extracts

PHY906 is comprised of a traditional hot water extract of four herbs, *Scutelleria baicalensis Georgi (S), Paeonia lactiflora Pall. (P), Glycyrrhiza uralensis Fisch.* (G), and *Ziziphus jujuba Mill (Z),* in the ratio of 3 : 2 : 2 : 2, respectively, and prepared under standard operational procedure. This extract comprises a complex mixture of multiple phytochemicals with multiple biological and pharmacological properties. At this time, it is not possible to identify the subset of relevant biologically active phytochemicals from the entire mixture. For this reason, we utilize high level chemical and biological metrics to characterize the PHY906 product.

The raw ingredients of PHY906 are pre-selected to meet rigid specifications set by PhytoCeutica for acceptance by the herbal manufacturer, Sun Ten Pharmaceuticals in Taiwan. The PHY906 extract is comprised of greater than 75% low molecular weight phytochemical compounds less than 1000 amu, 10% macromolecular components including protein, nucleic acid, complex carbohydrates, and 5% water. In addition, 10% by weight of excipient insoluble cellulose is added during the spray dry step in manufacturing. Heavy metals (Pb, Hg, Cd, As) are all less than 0.5 ppm, with mercury and cadmium less than 0.03 ppm, as detected by atomic absorption measurements. Pesticides levels (BHCs, DDTs, PCNB) are less than 0.2 ppm by LC-MS or GC-MS. Total bacteria counts are 260 cfu/g while E. coli and Salmonella species are not detected. Over 90% by weight of PHY906, excluding water content (5%) and insoluble starch excipient (10%), can be re-extracted. The final PHY906 liquid extract (100 mg / ml) is stable for 18 hours at room temperature and the properly stored bulk dry extract (vacuum packed, light tight and 4°C) appears to be stable for more than three years. More detailed information about PHY906 can be found in: Lam W, Bussom S, Guan F, Jiang Z, Zhang W, Gullen EA, Liu SH, Cheng YC, 2010, "The Four-Herb Chinese Medicine PHY906 Reduces Chemotherapy-Induced Gastrointestinal Toxicity", Sci. Transl. Med. 2(45):45ra59.

### Example 2: Subcutaneous melanoma tumor growth inhibition activity of PHY906 herbal extracts

The effect of PHY906 and anti-PD-L1 on the growth of subcutaneously implanted B 16 melanoma tumors in B6 mice was investigated.

The combination effect of PHY906 and PD-L1 on the growth of subcutaneously or brain implanted B 16 melanoma tumor in B6 mice was evaluated.

Both PHY906 (500 mg/kg, b.i.d, day 0 to day 5; FIG. 1C) and anti-PD-L1 (150 µg per animal, IP, day 0 and day 4; FIG. 1D) showed significant (P<0.005) inhibition on the growth of subcutaneously implant B16 melanoma tumor in B6 mice. Anti-PD-L1 plus PHY906 treatment showed greater inhibition on B16 tumor growth than anti-PD-L1 alone (FIGs. 1A and 1D).

Not all the animals responded to anti-PD-L1 treatment (FIG. 1D). However, anti-PD-L1 plus PHY906 combination treatment exhibited inhibitory effect on the growth of B16 tumors for all the animals (FIGs. 1A and 1E). In terms of animal toxicity, no weight loss and significant blood counts (white blood cells, red blood cells and platelets) were detected in any group of treatment (FIG. 1G).

In conclusion, PHY906 alone showed anti-melanoma effect *in vivo.* Further, anti-PD-L1 plus PHY906 combination treatment showed greater anti-melanoma activity than anti-PD-L1 alone.

### Example 3: Brain implanted melanoma tumor growth inhibition activity of PHY906 herbal extracts

The effect of PHY906 and anti-PD-L1 on the growth of brain implanted B16 melanoma tumors in B6 mice was investigated.

Brain metastasis is commonly found in melanoma. Thus, the combination effect of PHY906 and PD-L1 on the growth of the brain implanted B16 melanoma tumor in B6 mice was explored. Results indicated that PHY906 alone did not increase survival time for animals with B16 tumor brain implantation. Further, anti-PD-L1 alone did prolong the survival for animals with B16 tumor brain implantation. Luciferase measurement did show that anti-PD-L1 reduced the B16 tumor growth in the brain (FIGs. 2A-2E). The combination of anti-PD-L1 plus PHY906 did not change the median survival for animal carrying B16 tumor in brain as compared to anti-PD-L1 alone (FIG. 2A). For animal toxicity, no weight loss was detected in any group of treatment (FIG. 2F). In conclusion, PHY906 did not compromise the anti-B16 melanoma activity of anti-PD-L1 in the brain.

### Example 4: Subcutaneous melanoma tumor growth inhibition activity of PHY906 and S herbal extracts

The effect of isolated S, PHY906 and anti-PD-L1 on the growth of subcutaneously implanted B16 melanoma tumors in B6 mice was investigated.

The effects of the combination therapies of PHY906 plus anti-PD-L1, and S plus anti-PD-L1, on the growth of subcutaneously implant B16 melanoma tumor in B6 mice were tested. As shown in FIG. 3, S alone had comparable effect to PHY906 in terms of enhancing the anti-tumor activity of anti-PD-L1, and enhancing therapeutic activity of anti PD-L1.

### Example 5: Extract fractionation procedures

In certain embodiments of the invention, the extracts of the invention (which include water extracts and other solvent extracts) can be further processed into isolated fractions which can be used in the treatment methods of the invention. Non-limiting examples follow:

### Solid phase extract method

Herbal water extract or other solvent extract (1 ml, 100 mg/ml) is passed through a Discovery^{®} DSC-18 SPE Tube bed wt. 1 g. The bound chemicals are eluted sequentially using 1 ml water, 10% ethanol, 30% ethanol, 50% ethanol, 70% ethanol and 100% ethanol. The different fractions are dried *in vacuo* to form a pellet. The dried pellet is re-dissolved to 1 ml using water. The re-dissolved solution is regarded as a "100 mg/ml equivalent" of the original herbal water extract input for further experiments.

### Liquid chromatography-mass spectrometry (LC-MS)

The chromatographic separation is achieved on a ZORBAX-SBC18 column (100 mm × 2.1 mm i.d., 3.5µm, Agilent, Palo Alto, CA). Mobile phase A is water with 0.05% (v/v) formic acid and mobile phase B is acetonitrile. The liquid flow-rate is set at 0.3 mL/min, and the column temperature is maintained at 30 °C. The mobile phase consists of linear gradients of 0.05% (v/v) formic acid (A) and Acetonitrile (B). The chromatographic system used consists of an Agilent 1200 HPLC series, including a binary pump (Model G1312B), a vacuum degasser (Model G1379B), an autosampler (Model G1367C), and a column oven (Model G1316B). Masses of chemicals are detected by Applied Biosystems Sciex 4000 Q-trap^{®} mass spectrometer (Applied Biosystems Sciex; Foster, CA). Data acquisition is carried out by Analyst 1.4.2^{®} software on a DELL computer.

### Example 6: B16 melanoma growth inhibition activity of PHY906 with Anti-PD-L1, Anti-PD1 and Anti-CTLA4 antibodies

The effect of PHY906 alone and in combination with Anti-PD-L1, Anti-PD1 and Anti-CTLA4 antibodies against B16 melanoma, *in vivo,* was tested by administering a dosage of 500 mg/kg PHY906 twice per day (FIGs. 6A-6D), Anti-PD-L1 (FIG. 6A, 150µg / animal, once every 3 days), Anti-PD1 (FIGs. 6B / 6D, 150µg / animal, once every 3 days), Anti-CTLA4 (FIGs. 6C / 6D, 300µg / animal, once every 3 days) or a combination of two or more of these dosages.

The combination of Anti-PD-L1 and PHY906 had a large effect on hindering the growth of tumors in the mouse models (FIG. 6A). The combination of Ant-PD1 and PHY906 had a stronger effect than either individually as well. The combination of Anti-CTLA4 and PHY906 did not appear to have a strong effect on inhibiting tumor growth, however, the combination Ant-PD1, Anti-CTLA4 and PHY906 demonstrated a very strong effect, completely inhibiting growth of the tumor in certain subjects.

### Example 7: Hepa 1-6 mouse hepatic adenocarcinoma tumor inhibition activity of PHY906 with Anti-PD1 antibodies

The effect of PHY906 alone and in combination with Anti-PD1 antibodies against Hepa 1-6 mouse hepatic adenocarcinoma tumors, *in vivo,* was tested by administering: a dosage of 500 mg/kg PHY906 twice per day, a dosage of 70 or 200 µg of Anti-PD1 antibodies per mouse once at the beginning of the experiment or a combination of both treatments. It was found that individually treatment with either PHY906 or Anti-PD1 hindered tumor growth when compared to mice treated with a placebo. Further, treatment with a combination of both PHY906 and Anti-PD1 yielded a much stronger tumor inhibiting effect. The experiments were repeated and the efficacy of the combination treatment was reproducible.

### Example 8: CMT-167 non-small cell lung cancer tumor inhibition activity of PHY906 with Anti-PD1 antibodies

The effect of PHY906 alone and in combination with Anti-PD1 antibodies against CMT-167 non-small cell lung cancer tumors, *in vivo,* was tested by administering: a dosage of 500 mg/kg PHY906 twice per day, a dosage of 200 µg of Anti-PD1 antibodies per mouse once at the beginning of the experiment or a combination of both treatments. It was found that while both treatments individually hampered tumor growth, the combination of PHY906 and Anti-PD 1 demonstrated a much greater inhibitory effect on the growth of non-small cell lung cancer tumors.

## Claims

1. A kit comprising one or more immune checkpoint inhibitors selected from the group consisting of an anti-PD1, an anti-PD-L1 and an anti-CTLA4, and at least one herbal composition selected from the group consisting of:
(a) an herbal extract of *Scutellaria baicalensis (S);*
(b) the herbal extract PHY906, which comprises herbal extracts of *Scutellaria baicalensis (S), Glycyrrhiza uralensis (G), Paeonia lactiflora* (P), and *Ziziphus jujuba* (Z) in a 3 : 2 : 2 : 2 ratio of S, G, P and Z;
(c) an herbal extract comprising an extract of S; and
(d) an herbal extract comprising a combination of S, G, P and Z.

2. The kit of claim 1, further comprising one or more pharmaceutically acceptable carriers.

3. A herbal composition for use in a method of treating cancer in a subject, wherein the herbal composition is at least one herbal composition selected from the group consisting of:
(a) an herbal extract of *Scutellaria baicalensis (S);*
(b) the herbal extract PHY906, which comprises herbal extracts of *Scutellaria baicalensis (S), Glycyrrhiza uralensis (G), Paeonia lactiflora* (P), and *Ziziphus jujuba* (Z) in a 3 : 2 : 2 : 2 ratio of S, G, P and Z;
(c) an herbal extract comprising an extract of S; and
(d) an herbal extract comprising a combination of S, G, P and Z;
wherein further one or more immune checkpoint inhibitors selected from the group consisting of an anti-PD1, an anti-PD-L1 and an anti-CTLA4 is/are to be administered to the subject,
wherein the cancer is at least one selected from the group consisting of melanoma, non-small cell lung cancer, renal cell carcinoma, liver cancer, colon cancer, urothelial bladder cancer and pancreatic cancer.

4. The kit of claim 1 or the herbal composition for use according to claim 3, wherein (a) or (c) further comprises an herbal extract, which is derived from at least one herb selected from the group consisting of *Glycyrrhiza uralensis (G), Paeonia lactiflora* (P), and *Ziziphus jujuba (Z).*

5. The kit of claim 1 or the herbal composition for use according to claim 3, wherein the immune checkpoint inhibitor is selected from the group consisting of Ipilimumab, Pembrolizumab, Nivolumab, Durvalumab and Atezolizumab.

6. The herbal composition for use according to claim 3 or 4, wherein the herbal composition minimizes or eliminates one or more of the immune checkpoint inhibitors' gastro-intestinal side effects selected from the group consisting of nausea, vomiting, fatigue, colitis and diarrhea.

7. The herbal composition for use according to claim 3 or 4, wherein the cancer is melanoma.

8. The herbal composition for use according to claim 3 or 4, wherein the herbal composition is to be administered to the subject orally, preferably the herbal composition is administered to the subject orally in a form selected from the group consisting of a pill, tablet, capsule, soup, tea, concentrate, dragees, liquids, drops, and gelcaps.

9. The herbal composition for use according to claim 3 or 4, wherein the therapeutically effective amount of the herbal composition to be administered is 20 mg/day to 2 g/day.

10. The herbal composition for use according to claim 9, wherein the therapeutically effective amount of the herbal composition to be administered is 1,600 mg/day.

11. The herbal composition for use according to claim 3 or 4, wherein the herbal composition is to be administered twice daily.

12. The herbal composition for use according to claim 11, wherein the herbal composition is to be administered twice daily for one to two weeks, followed by a suspension of treatment for at least one week.

13. The herbal composition for use according to claim 3 or 4, wherein the herbal composition is to be administered at a time selected from prior to, simultaneously with and after administration of the one or more immune checkpoint inhibitors to the subject.

14. The herbal composition for use according to claim 3, wherein the subject is a mammal, preferably the mammal is a human.

## Patentansprüche

1. Ein Kit, das einen oder mehrere Immun-Checkpoint-Inhibitoren, ausgewählt aus der Gruppe, bestehend aus einem Anti-PD1, einem Anti-PD-L1 und einem Anti-CTLA4, und mindestens eine pflanzliche Zusammensetzung, ausgewählt aus der Gruppe, bestehend aus:
(a) einem Pflanzenextrakt von *Scutellaria baicalensis (S);*
(b) dem Pflanzenextrakt PHY906, der Pflanzenextrakte von *Scutellaria baicalensis (S), Glycyrrhiza uralensis (G), Paeonia lactiflora* (P) und *Ziziphus jujuba* (Z) in einem Verhältnis von S, G, P und Z von 3 : 2 : 2 : 2 umfasst;
(c) ein Pflanzenextrakt, der einen Extrakt von S umfasst; und
(d) ein Pflanzenextrakt, der eine Kombination von S, G, P und Z umfasst.

2. Kit nach Anspruch 1, das ferner einen oder mehrere pharmazeutisch verträgliche Träger umfasst.

3. Pflanzliche Zusammensetzung zur Verwendung in einem Verfahren zur Behandlung von Krebs bei einem Subjekt, wobei die pflanzliche Zusammensetzung mindestens eine pflanzliche Zusammensetzung ist, ausgewählt aus der Gruppe bestehend aus:
(a) einem Pflanzenextrakt von *Scutellaria baicalensis (S);*
(b) dem Pflanzenextrakt PHY906, der Pflanzenextrakte von *Scutellaria baicalensis (S), Glycyrrhiza uralensis (G), Paeonia lactiflora* (P) und *Ziziphus jujuba* (Z) in einem Verhältnis von S, G, P und Z von 3:2:2:2 umfasst;
(c) ein Pflanzenextrakt, der einen Extrakt von S umfasst; und
(d) ein Pflanzenextrakt, der eine Kombination von S, G, P und Z umfasst;
wobei ferner ein oder mehrere Immun-Checkpoint-Inhibitoren, ausgewählt aus der Gruppe, bestehend aus einem Anti-PD1, einem Anti-PD-L1 und einem Anti-CTLA4 an das Subjekt zu verabreichen sind,
wobei der Krebs mindestens einer ist, der aus der Gruppe ausgewählt ist, die aus Melanom, nicht-kleinzelligem Lungenkrebs, Nierenzellkarzinom, Leberkrebs, Dickdarmkrebs, Urothelkarzinom der Blase und Bauchspeicheldrüsenkrebs besteht.

4. Kit nach Anspruch 1 oder pflanzliche Zusammensetzung zur Verwendung nach Anspruch 3, wobei (a) oder (c) ferner einen Pflanzenextrakt umfasst, der von mindestens einer Pflanze, ausgewählt aus der Gruppe, bestehend aus *Glycyrrhiza uralensis (G), Paeonia lactiflora* (P) und *Ziziphus jujuba* (Z) stammt.

5. Kit nach Anspruch 1 oder pflanzliche Zusammensetzung zur Verwendung nach Anspruch 3, wobei der Immun-Checkpoint-Inhibitor aus der Gruppe bestehend aus Ipilimumab, Pembrolizumab, Nivolumab, Durvalumab und Atezolizumab ausgewählt ist.

6. Die pflanzliche Zusammensetzung zur Verwendung gemäß Anspruch 3 oder 4, wobei die pflanzliche Zusammensetzung eine oder mehrere der gastrointestinalen Nebenwirkungen der Immun-Checkpoint-Inhibitoren, ausgewählt aus der Gruppe bestehend aus Übelkeit, Erbrechen, Müdigkeit, Kolitis und Durchfall minimiert oder eliminiert.

7. Die pflanzliche Zusammensetzung zur Verwendung gemäß Anspruch 3 oder 4, wobei der Krebs ein Melanom ist.

8. Die pflanzliche Zusammensetzung zur Verwendung gemäß Anspruch 3 oder 4, wobei die pflanzliche Zusammensetzung dem Subjekt oral zu verabreichen ist, wobei die pflanzliche Zusammensetzung dem Subjekt vorzugsweise oral in einer Form, ausgewählt aus der Gruppe, bestehend aus einer Pille, Tablette, Kapsel, Suppe, Tee, Konzentrat, Dragees, Flüssigkeiten, Tropfen und Gelkapseln verabreicht wird.

9. Die pflanzliche Zusammensetzung zur Verwendung gemäß Anspruch 3 oder 4, wobei die zu verabreichende therapeutisch wirksame Menge der pflanzlichen Zusammensetzung 20 mg/Tag bis 2 g/Tag beträgt.

10. Die pflanzliche Zusammensetzung zur Verwendung gemäß Anspruch 9, wobei die zu verabreichende therapeutisch wirksame Menge der pflanzlichen Zusammensetzung 1600 mg/Tag beträgt.

11. Die pflanzliche Zusammensetzung zur Verwendung gemäß Anspruch 3 oder 4, wobei die pflanzliche Zusammensetzung zweimal täglich zu verabreichen ist.

12. Die pflanzliche Zusammensetzung zur Verwendung gemäß Anspruch 11, wobei die pflanzliche Zusammensetzung zweimal täglich für ein bis zwei Wochen zu verabreichen ist, gefolgt von einer Aussetzung der Behandlung für mindestens eine Woche.

13. Die pflanzliche Zusammensetzung zur Verwendung gemäß Anspruch 3 oder 4, wobei die pflanzliche Zusammensetzung zu einem Zeitpunkt zu verabreichen ist, der aus vor, gleichzeitig mit und nach der Verabreichung des einen oder der mehreren Immun-Checkpoint-Inhibitoren an das Subjekt ausgewählt ist.

14. Die pflanzliche Zusammensetzung zur Verwendung gemäß Anspruch 3, wobei das Subjekt ein Säugetier ist, vorzugsweise ist das Säugetier ein Mensch.

## Revendications

1. Kit comprenant un ou plusieurs inhibiteurs des points de contrôle immunitaire choisis dans le groupe constitué d'un anti-PD1, d'un anti-PD-L1 et d'un anti-CTLA4, et au moins une composition à base de plantes choisie dans le groupe constitué de :
(a) un extrait végétal de *Scutellaria baicalensis (S)* ;
(b) l'extrait végétal PHY906, qui comprend des extraits végétaux de *Scutellaria baicalensis (S), Glycyrrhiza uralensis (G), Paeonia lactiflora* (P) et *Ziziphus jujuba* (Z) dans un rapport 3 : 2 : 2 : 2 : 2 de S, G, P et Z ;
(c) un extrait végétal comprenant un extrait de S ; et
(d) un extrait végétal comprenant une combinaison de S, G, P et Z.

2. Kit selon la revendication 1, comprenant en outre un ou plusieurs supports pharmaceutiques acceptables.

3. Composition à base de plantes à utiliser dans un procédé de traitement du cancer chez un sujet, dans laquelle
la composition à base de plantes est au moins une composition à base de plantes choisie dans le groupe constitué par :
(a) un extrait végétal de *Scutellaria baicalensis (S)* ;
(b) l'extrait végétal PHY906, qui comprend des extraits végétaux de *Scutellaria baicalensis (S), Glycyrrhiza uralensis (G), Paeonia lactiflora* (P) et *Ziziphus jujuba* (Z) dans un rapport 3 : 2 : 2 : 2 : 2 de S, G, P et Z ;
(c) un extrait végétal comprenant un extrait de S ; et
(d) un extrait végétal comprenant une combinaison de S, G, P et Z ;
dans lequel un ou plusieurs inhibiteurs des points de contrôle immunitaire choisis dans le groupe constitué d'un anti-PD1, d'un anti-PD-L1 et d'un anti-CTLA4 est/sont administré(s) au sujet,
dans lequel le cancer est au moins l'un des cancers suivants : mélanome, cancer du poumon non à petites cellules, carcinome rénal, cancer du foie, cancer du côlon, cancer urothélial de la vessie et cancer du pancréas.

4. Kit selon la revendication 1 ou composition à base de plantes à utiliser selon la revendication 3, dans lequel (a) ou (c) comprend en outre un extrait de plantes, qui est dérivé d'au moins une plante sélectionnée parmi le groupe constitué par *Glycyrrhiza uralensis (G), Paeonia lactiflora* (P) et *Ziziphus jujuba (Z).*

5. Kit selon la revendication 1 ou composition à base de plantes à utiliser selon la revendication 3, dans lequel l'inhibiteur de point de contrôle immunitaire est choisi dans le groupe constitué par le Ipilimumab, le Pembrolizumab, le Nivolumab, le Durvalumab et l'Atezolizumab.

6. Composition à base de plantes à utiliser selon les revendications 3 ou 4, dans laquelle la composition à base de plantes minimise ou élimine un ou plusieurs des effets secondaires gastro-intestinaux des inhibiteurs des points de contrôle immunitaire choisis dans le groupe constitué des nausées, des vomissements, de la fatigue, de la colite et de la diarrhée.

7. Composition à base de plantes à utiliser selon les revendications 3 ou 4, dans laquelle le cancer est un mélanome.

8. Composition à base de plantes à utiliser selon les revendications 3 ou 4, dans laquelle la composition à base de plantes doit être administrée au sujet par voie orale, de préférence sous une forme choisie dans le groupe constitué d'une pilule, d'un comprimé, d'une capsule, d'une soupe, d'un thé, d'un concentré, de dragées, de liquides, de gouttes et de gélules.

9. Composition à base de plantes à utiliser selon les revendications 3 ou 4, dans laquelle la quantité thérapeutiquement efficace de la composition à base de plantes à administrer est comprise entre 20 mg/jour et 2 g/jour.

10. Composition à base de plantes à utiliser selon la revendication 9, dans laquelle la quantité thérapeutiquement efficace de la composition à base de plantes à administrer est de 1 600 mg/jour.

11. Composition à base de plantes à utiliser selon les revendications 3 ou 4, dans laquelle la composition à base de plantes doit être administrée deux fois par jour.

12. Composition à base de plantes à utiliser selon la revendication 11, dans laquelle la composition à base de plantes doit être administrée deux fois par jour pendant une à deux semaines, suivie d'une suspension du traitement pendant au moins une semaine.

13. Composition à base de plantes à utiliser selon les revendications 3 ou 4, dans laquelle la composition à base de plantes doit être administrée à un moment donné, à savoir : avant, simultanément et après l'administration d'un ou de plusieurs inhibiteurs des points de contrôle immunitaire au sujet.

14. Composition à base de plantes à utiliser selon la revendication 3, dans laquelle le sujet est un mammifère, de préférence un être humain.
